(19) Europäisches Patentamt
European Patent Office
Office européen des brevets

(11) Numéro de publication: **0 389 338 A1**

(12) **DEMANDE DE BREVET EUROPEEN**

(21) Numéro de dépôt: **90400703.6**

(51) Int. Cl.[5]: **C07F 15/00, A61K 31/28**

(22) Date de dépôt: **16.03.90**

(30) Priorité: **17.03.89 FR 8903511**

(43) Date de publication de la demande: **26.09.90 Bulletin 90/39**

(84) Etats contractants désignés: **AT BE CH DE DK ES FR GB GR IT LI LU NL SE**

(71) Demandeur: **Société anonyme dite: LABORATOIRE ROGER BELLON**
**159, avenue A. Peretti**
**F-92201 Neuilly-sur-Seine(FR)**

(72) Inventeur: **Barreau, Michel**
**24bis, Avenue du Clos de Sénart**
**F-91230 Montgeron(FR)**
Inventeur: **Chottard, Jean Claude**
**1 à 5, Avenue de la Source**
**F-94130 Nogent s/Marne(FR)**
Inventeur: **Le Pecq, Jean-Bernard**
**37 Square St. Charles**
**F-75012 Paris(FR)**
Inventeur: **Mailliet, Patrick**
**87, Rue Delayrac**
**F-94120 Fontenay-Sous-Bois(FR)**

(74) Mandataire: **Savina, Jacques et al**
**RHONE-POULENC SANTE, Service Brevets,**
**20 Avenue Raymond Aron**
**F-92165 Antony Cédex(FR)**

(54) **Nouveaux complexes dérivés du platine, leur préparation et les compositions pharmaceutiques qui les contiennent.**

(57) Nouveaux complexes de formule générale (I) dans laquelle

- $R_1$ et $R_2$ forment ensemble un carbocycle polycyclique saturé ou insaturé contenant 7 à 12 atomes de carbone, ou un hétérocycle saturé ou partiellement saturé, mono, bi ou tricyclique contenant 5 à 11 chaînons et dont l'hétéroatome est choisi parmi l'oxygène, le soufre ou l'azote, ce dernier pouvant être éventuellement substitué par un radical alcoyloxycarbonyle et,
- $X_1$ et $X_2$ représentent des atomes de chlore ou forment ensemble
- soit un radical de structure :

$$-O-\underset{\overset{\|}{O}}{C}-(CR_6R_7)_n-\underset{\overset{\|}{O}}{C}-O \qquad (II)$$

dans laquelle n égale 0 à 2 et $R_6$ et $R_7$ identiques ou différents sont des atomes d'hydrogène ou lorsque n égale 1 peuvent être des radicaux alcoyle ou former ensemble avec l'atome de carbone auquel ils sont attachés un radical cyclobutyle,
- soit un radical de structure :

$$-O-\underset{\overset{\|}{O}}{C}-(CR_6R_7)_n-\underset{\overset{\|}{O}}{\overset{\overset{OH}{|}}{P}}-O- \qquad (III)$$

dans laquelle n, $R_6$ et $R_7$ sont définis comme ci-dessus, ou leurs sels lorsqu'ils existent, et leurs hydrates, leur préparation et les compositions pharmaceutiques qui les contiennent.

EP 0 389 338 A1

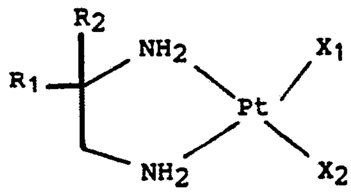

(I)

# NOUVEAUX COMPLEXES DERIVES DU PLATINE, LEUR PREPARATION ET LES COMPOSITIONS PHARMACEUTIQUES QUI LES CONTIENNENT

La présente invention concerne de nouveaux complexes dérivés du platine, de formule générale :

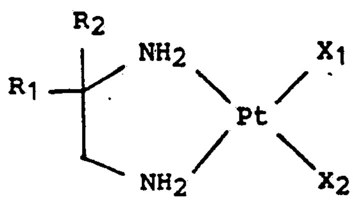

(I)

éventuellement leurs sels métalliques et leurs hydrates, leur préparation et les compositions pharmaceutiques qui les contiennent.

Dans la demande de brevet européen 290 169 ont été décrits des complexes du platine de formule générale :

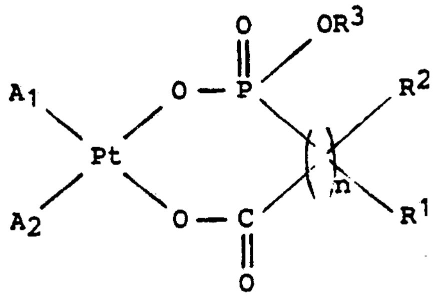

dans laquelle :

- $A_1$ et $A_2$ forment ensemble un radical de structure :

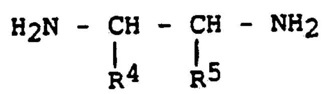

- $R^4$ et $R^5$ pouvant être des atomes d'hydrogène ou des radicaux alcoyle éventuellement substitués,
- $R^1$ et $R^2$ étant notamment des atomes d'hydrogène ou des radicaux alcoyle éventuellement substitués,
- $R^3$ étant notamment l'atome d'hydrogène ou un cation et
- n étant 0 à 2.

Ces complexes sont doués d'activité antitumorale.

Dans le brevet belge 900 842 ont été décrits des complexes diaminés du platine ayant une activité antitumorale et répondant à la formule générale :

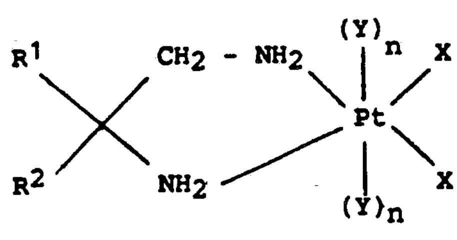

dans laquelle $R^1$ et $R^2$ forment ensemble un radical alcoylène contenant 3 à 6 atomes de carbone et X représente notamment un atome d'halogène, ou les 2 radicaux X forment ensemble des radicaux -OCO-

COO- ou $-OCO-CHR_3-COO-$.

Il a été trouvé maintenant, que les complexes de formule générale (I) dans laquelle :

- $R_1$ et $R_2$ forment ensemble un carbocycle polycyclique saturé ou insaturé et contenant 7 à 12 atomes de carbone, ou un hétérocycle saturé ou partiellement saturé, mono, bi ou tricyclique contenant 5 à 11 chaînons et un hétéroatome choisi parmi l'azote, l'oxygene ou le soufre et dont l'atome d'azote est éventuellement substitué par un radical alcoyloxycarbonyle et,
- $X_1$ et $X_2$ représentent des atomes de chlore ou forment ensemble
- soit un radical de structure :

$$-O-\underset{\underset{O}{\parallel}}{C}-(CR_6R_7)_n-\underset{\underset{O}{\parallel}}{C}-O- \qquad (III)$$

dans laquelle n égale 0 à 2 et $R_6$ et $R_7$ identiques ou différents sont des atomes d'hydrogène ou lorsque n=1 peuvent être des radicaux alcoyle ou former ensemble avec l'atome de carbone auquel ils sont attachés, un radical cyclobutyle,

- soit un radical de structure :

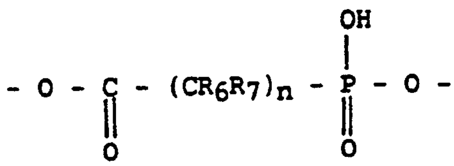

(IV)

dans laquelle n, $R_6$ et $R_7$ sont définis comme ci-dessus,

ainsi que leurs sels lorsqu'ils existent, et leurs hydrates, manifestent des propriétés antitumorales particulièrement intéressantes.

Dans la définition ci-dessus, les radicaux alcoyle et alcènyle sont droits ou ramifiés et contiennent 1 à 4 atomes de carbone, les atomes d'halogène peuvent être choisis parmi le chlore, le brome, le fluor et l'iode.

Lorsque $R_1$ et $R_2$ forment ensemble un carbocycle polycyclique, à titre d'exemple, ce dernier peut être choisi parmi : bicyclo (2.2.1) heptane, bicyclo (3.2.1) octane, bicyclo (2.2.2) octane, bicyclo (3.2.2) nonane, bicyclo (3.3.1) nonane, adamantane, décahydronaphtalène, tétrahydronaphtalène, spiro (5.5) undécane, tricyclo (5.2.1.0/2,6) décane ou indane.

Lorsque $R_1$ et $R_2$ forment ensemble un hétérocycle saturé ou partiellement saturé, à titre d'exemple, ce dernier peut être choisi parmi : chromanyle-4, coumaryle-3, homopipéridinyle-4, pipéridinyle-4, pyrrolidinyle-3, quinuclidinyle-3, tétrahydropyrannyle-4, tétrahydrofurannyle-3, tétrahydrothiopyrannyle-4, tétrahydrothiofurannyle-3 ou tétrahydroquinoléyle-4.

A/ Selon l'invention, les complexes de formule générale (I) peuvent être obtenus par action du tétrachloroplatinate de potassium sur une diamine de formule générale :

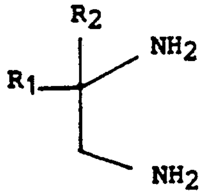

(V)

dans laquelle $R_1$ et $R_2$ sont définis comme précédemment, suivie éventuellement, lorsque l'on veut obtenir un complexe de formule générale (I) pour lequel $X_1$ et $X_2$ sont des radicaux de formule générale (III) ou (IV) de la transformation du complexe chloré obtenu en un complexe dicarboxylé ou phosphonocarboxylé.

L'action du tétrachloroplatinate de potassium s'effectue généralement sous azote et à l'abri de la lumière sur la diamine éventuellement libérée in situ de son sel, en milieu aqueux ou hydroalcoolique (éthanol-eau par exemple, dans des proportions pouvant aller jusqu'à 20-80 en volumes), à une température comprise entre 0 et 80° C.

La transformation du complexe chloré en un complexe dicarboxylé ou phosphonocarboxylé s'effectue par l'intermédiaire du complexe dinitrate de diaqua de structure :

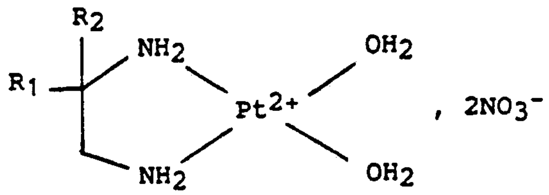

(VI)

dans laquelle $R_1$ et $R_2$ sont définis comme précédemment, qui est ensuite transformé en complexe dicarboxylé ou phosphonocarboxylé soit par action directe d'un sel d'un acide de formule générale :

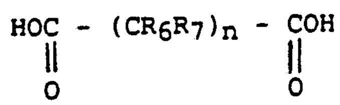

(VIIa)

ou

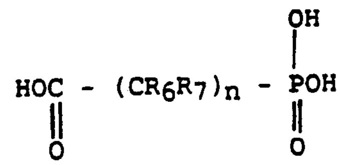

(VIIb)

dans laquelle $R_6$, $R_7$ et n sont définis comme précédemment, soit par passage sur une résine échangeuse d'anions (forme OH) suivi de l'addition d'un acide de formule générale (VIIa) ou (VIIb).

Le complexe de formule générale (VI) peut être obtenu par action du nitrate d'argent sur le complexe dichloré, en milieu aqueux ou dans un mélange alcool-eau (éthanol-eau ou méthanol-eau par exemple) dans des proportions pouvant varier, de préférence, jusqu'à 10 % de méthanol, en opérant sous azote et à l'abri de la lumière à une température comprise entre 20 et 70° C.

L'action directe d'un sel de l'acide de formule générale (VIIa) ou (VIIb) sur le complexe de formule générale (VI) s'effectue en milieu aqueux ou hydroalcoolique, à l'abri de la lumière et sous atmosphère d'azote, à une temperature comprise entre O et 50° C.

Il n'est pas indispensable d'isoler le complexe de formule générale (VI) pour le mettre en oeuvre dans cette réaction.

Le sel de l'acide de formule générale (VIIa) ou (VIIb) est choisi, de préférence, parmi les sels alcalins (sel de sodium ou de potassium par exemple) ; il peut être formé in situ au cours de la réaction.

.Dans ces conditions, le complexe de formule générale (I) pour lequel $X_1$ et $X_2$ forment un radical de formule générale (IV), est obtenu à l'état de sel alcalin qui peut être libéré et éventuellement transformé en un autre sel selon les méthodes connues.

Lorsque l'on transforme le complexe de formule générale (VI) par passage sur résine échangeuse d'anion, on opère généralement par percolation de la solution de complexe dinitrate de diaqua au travers d'une colonne de 1,2 à 1,5 fois la quantité théorique de résine Amberlite IRA 402 (forme OH) à l'abri de la lumière.

L'addition de l'acide de formule générale (VIIa) ou (VIIb) s'effectue en milieu aqueux, à l'abri de la lumière et sous atmosphère d'azote, à une température comprise entre 0 et 50° C.

B/ Selon l'invention, les produits de formule générale (I) peuvent également être obtenus par l'intermédiaire du complexe diiodé de formule générale :

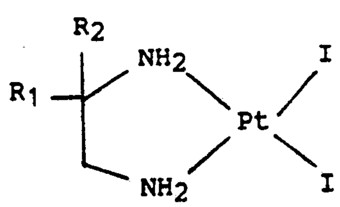

(VIII)

dans laquelle $R_1$ et $R_2$ sont définis comme précédemment, par action de tétrachloroplatinate de potassium sur une diamine de formule générale (V), en présence d'un excès d'iodure de potassium. Le complexe diiodé obtenu est ensuite traité par le nitrate d'argent puis par un chlorure alcalin en excès, puis éventuellement lorsque l'on veut obtenir un complexe de formule générale (I) pour lequel $X_1$ et $X_2$ sont des radicaux de formule générale (III) ou (IV), on transforme le complexe chloré obtenu en un complexe dicarboxylé ou phosphonocarboxylé.

La préparation du complexe diiodé intermédiaire s'effectue dans des conditions identiques à celles décrites précédemment pour la préparation d'un complexe chloré du platine de formule générale (I) par action du tétrachloroplatinate de potassium sur une amine de formule générale (V).

L'addition du nitrate d'argent s'effectue dans des conditions analogues à celles décrites précédemment en A/.

Le chlorure alcalin peut être choisi parmi le chlorure de sodium ou de potassium ; il n'est pas nécessaire d'isoler le produit intermédiairement formé pour procéder à cette addition. La réaction peut être effectuée à une température comprise entre 0 et 70 °C.

La transformation du complexe dichloré de formule générale (I) en un complexe dicarboxylé ou phosphonocarboxylé s'effectue le cas échéant dans les conditions décrites précédemment en A/.

C/ Selon l'invention, les produits de formule générale (I) peuvent aussi être obtenus par action du trichloro(éthylène)platinate de potassium sur une diamine de formule générale (V), suivie éventuellement, lorsque l'on veut obtenir un produit de formule générale (I) dans laquelle $X_1$ et $X_2$ sont des radicaux de formule générale (III) ou (IV), de la transformation du complexe chloré obtenu en un complexe dicarboxylé ou phosphonocarboxylé.

La réaction s'effectue généralement sur la diamine (éventuellement libérée in situ de son sel), dans un alcool ou en milieu hydroalcoolique, à une température comprise entre O et 40 °C.

La transformation du complexe dichloré obtenu en un produit de formule générale (I) dans laquelle $X_1$ et $X_2$ sont des radicaux de formule générale (III) ou (IV) s'effectue dans les conditions décrites précédemment en A/.

Les diamines de formule générale (V) peuvent être préparées comme décrit ci-après dans les exemples, notamment par réaction de Strecker en présence de cyanure de sodium ou de potassium et de chlorure d'ammonium, d'ammoniac ou de benzylamine, sur une cétone de formule générale :

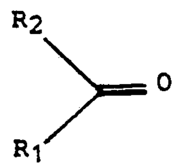

(XVI)

dans laquelle $R_1$ et $R_2$ sont définis comme ci-dessus, suivie de l'hydrogénation catalytique de l'aminonitrile formé, et, le cas échéant, de l'élimination du ou des radicaux protecteurs.

La réaction de Strecker s'effectue dans l'eau ou dans un alcool (méthanol, éthanol par exemple) ou en milieu hydroalcoolique, à une température comprise entre 20 et 80 °C.

La réaction de Strecker peut aussi être mise en oeuvre en présence de cyanure de triméthylsilyle sur une cétone de formule générale (XVI), en milieu anhydre et éventuellement en présence d'une quantité catalytique d'acide de Lewis.

Dans ce cas, la réaction s'effectue avec ou sans solvant, à une température comprise entre -20 et + 40 °C. Le cas échéant, l'acide de Lewis peut être choisi parmi l'iodure de zinc ou le chlorure de cobalt ; le solvant peut être choisi parmi l'éther éthylique ou propylique, le tétrahydrofuranne, le dichlorométhane ou le chloroforme.

La cyanhydrine triméthylsilylée ainsi obtenue est traitée par l'ammoniac ou la benzylamine en solution dans un alcool (par exemple le méthanol ou l'éthanol), à une température comprise entre 20 °C et la température de reflux du mélange réactionnel. Cette méthode de mise en oeuvre de la réaction de Strecker est plus spécialement choisie dans le cas de cétones de formule générale (XVI) très encombrées (comme par exemple, les cétones pour lesquelles $R_1$ et $R_2$ forment ensemble un cycle bicyclo(2.2.2)octane bicyclo-(3.2.2)nonane ou une quinuclidine).

Dans les réactions décrites ci-dessus, il est entendu que, selon les réactifs employés, l'aminonitrile obtenu intermédiairement peut être protégé sur l'amine.

L'hydrogénation peut être effectuée indifféremment sur l'aminonitrile dont la fonction amine est libre ou protégée. Il est également possible de protéger la fonction amine préalablement à l'hydrogénation catalytique. Dans ce cas, la protection s'effectue notamment par acylation (par exemple selon Org. Synth., vol.IV, p. 5 et 6).

L'hydrogénation catalytique de l'aminonitrile s'effectue généralement sous une pression de 100 à 4000 kPa en présence d'un catalyseur tel que le platine d'Adams, le nickel de Raney ou le rhodium sur alumine, à une température comprise entre 20 et 80 °C, et l'on opère dans un alcool (éthanol ou méthanol par exemple) soit en présence d'acide chlorhydrique, soit en milieu basique (ammoniac par exemple), ou bien

dans l'acide acétique en présence d'anhydride acétique.

Lorsque la réduction est effectuée en milieu acétique, il est nécessaire d'éliminer les groupements acétyle de la diamine obtenue. On opère par traitement acide postérieurement à l'hydrogénation.

Dans le cas des cétones de formule générale (XVI) pour lesquelles la fonction cétone ne se trouve pas dans un plan de symétrie de la molécule, la réaction de Strecker conduit à un mélange des racémiques exocyclique et endocyclique dans lequel le racémique exocyclique est majoritaire.

Il est entendu que les dérivés du platine de formule générale (I) préparés à partir des diamines exocycliques ou endocycliques ou de leurs mélanges entrent dans le cadre de la présente invention.

Les diamines de formule générale (V) de forme racémique exocyclique pure peuvent être obtenues par recristallisations successives de l'aminonitrile intermédiaire.

Les diamines de formule générale (V) de forme racémique endo pure peuvent être obtenues à partir des aminoacides endo correspondants de formule générale :

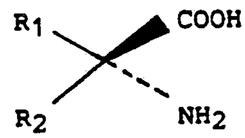

(XVII)

dans laquelle $R_1$ et $R_2$ sont définis comme ci-dessus, et dont la fonction amine est préalablement protégée, par transformation de la fonction acide en un amide selon les méthodes connues, puis élimination du radical protecteur d'amine et réduction de l'amide par action du diborane dans les conditions décrites dans J. Org. Chem., 38(16), 2786 (1973) ou J. Org. Chem., 42(16), 3153 (1982).

Les aminoacides de formule générale (XVII) peuvent être obtenus à partir des cétones de formule générale (XVI) pour lesquelles $R_1$ et $R_2$ sont définis comme ci-dessus, par action du carbonate d'ammonium et d'un cyanure alcalin, en milieu hydroalcoolique à une température comprise entre 50 et 100° C, puis par hydrolyse de la spirohydantoïne ainsi obtenue, par action d'une solution aqueuse d'hydroxyde de baryum, en autoclave à une température comprise entre 120 et 200° C.

Les nouveaux complexes selon l'invention pour lesquels $X_1$ et $X_2$ forment ensemble un radical de formule générale (IV) peuvent être transformés en sels métalliques. Ces sels peuvent être obtenus par action d'une base métallique (par exemple alcaline ou alcalino-terreuse) dans un solvant approprié.

Comme exemples de sels pharmaceutiquement acceptables peuvent être cités les sels avec les métaux alcalins (sodium, potassium, lithium) ou les sels avec les métaux alcalino-terreux (magnésium, calcium).

Les nouveaux complexes selon la présente invention peuvent être éventuellement purifiés par des méthodes physiques telles que la cristallisation ou la chromatographie.

Les produits de formule générale (I) ainsi que leurs sels pharmaceutiquement acceptables et leurs hydrates sont particulièrement utiles pour le traitement des tumeurs malignes.

Ils se sont montrés particulièrement actifs sur les tumeurs greffées de la souris : ils sont notamment actifs à des doses comprises entre 4 et 120 mg/kg par voie intrapéritonéale sur la leucémie L1210. Leur dose maximale tolérée est comprise entre 10 mg/kg et des doses supérieures à 120 mg/kg par voie intrapéritonéale chez la souris.

D'un intérêt particulier, sont les produits de formule générale (I) pour lesquels $R_1$ et $R_2$ forment ensemble un carbocycle polycyclique choisi parmi : bicyclo(2.2.1)heptane, adamantane, bicyclo(3.2.1)-octane, bicyclo(2.2.2)octane ou tricyclo(5.2.1.0/2,6)décane et $X_1$ et $X_2$ représentent des atomes de chlore ou un radical de structure (IV) dans laquelle $R_6$ et $R_7$ sont des atomes d'hydrogène ou des radicaux méthyle.

Et parmi ces produits, plus spécialement intéressants sont les produits suivants :

- cis (amino-2 aminométhyl-2 bicyclo (2.2.1) heptane) dichloro platine,
- cis (amino-2 aminométhyl-2 bicyclo (3.2.1) octane) dichloro platine,
- cis (amino-2 aminométhyl-2 adamantane) dichloro platine,
- cis (amino-3 aminométhyl-3 bicyclo (3.2.1) octane) dichloro platine,
- cis (amino-2 aminométhyl-2 bicyclo (2.2.2) octane) dichloro platine.

Les exemples suivants donnés à titre non limitatif illustrent la présente invention :

EXEMPLE 1

On dissout, sous atmosphère d'azote et à l'abri de la lumière, 1,49 g de dichlorhydrate d'amino-2 aminométhyl-2 bicyclo (2.2.1) heptane, sous forme du mélange des racémiques exo et endo dans un rapport (4/1), dans 6 $cm^3$ de méthanol et 14 $cm^3$ d'une solution 1 N d'hydroxyde de sodium dans l'eau. A la solution opalescente obtenue, on ajoute, sous agitation en 10 minutes, 2,84 g de tétrachloroplatinate de potassium en solution dans 14 $cm^3$ d'eau.

L'agitation est poursuivie pendant 20 heures à 20° C. Le précipité formé est essoré, lavé par 3 fois 5 $cm^3$ d'eau et séché sous pression réduite (2,5 kPa) en présence d'anhydride phosphorique. On obtient ainsi 2,48 g d'hydrate de cis (amino-2 aminométhyl-2 bicyclo (2.2.1) heptane) dichloro platine, mélange des racémiques exo et endo dans un rapport (4/1), sous forme d'une poudre jaune-beige qui fond avec décomposition à 331° C.

Le dichlorhydrate d'amino-2 aminométhyl-2 bicyclo (2.2.1) heptane, mélange des racémiques exo et endo dans un rapport (4/1), peut être obtenu de la manière suivante :

On porte à reflux 3 g d'acétylamino-2 acétylaminométhyl-2 bicyclo (2.2.1) heptane, sous forme du mélange des racémiques exo et endo dans un rapport (4/1), dans 100 $cm^3$ d'une solution 6 N d'acide chlorhydrique dans l'eau pendant 8 heures. On concentre à sec sous pression réduite (5,2 kPa), on reprend le résidu d'évaporation par 50 $cm^3$ d'éthanol et on ajoute, goutte à goutte, 150 $cm^3$ d'éther isopropylique. Le précipité obtenu est essoré sur verre fritté, lavé par 20 $cm^3$ d'un mélange d'éthanol et d'éther isopropylique (25-75 en volumes) puis par 2 fois 20 $cm^3$ d'éther isopropylique et enfin séché en étuve à 80° C. On obtient ainsi 2,53 g de dichlorhydrate d'amino-2 aminométhyl-2 bicyclo (2.2.1) heptane sous forme de cristaux blancs qui fondent à 262-4° C.

L'acétylamino-2 acétylaminométhyl-2 bicyclo (2.2.1) heptane, mélange des racémiques exo et endo dans un rapport (4/1), peut être obtenu de la manière suivante :

On charge dans un autoclave de 250 $cm^3$ une solution de 6,05 g d'acétylamino-2 cyano-2 bicyclo (2.2.1) heptane, sous forme du mélange des racémiques exo et endo dans un rapport (4/1), dans 100 $cm^3$ d'acide acétique et on ajoute 6,4 $cm^3$ d'anhydride acétique et 0,6 g d'oxyde de platine d'Adams. On soumet à l'hydrogénation catalytique, à 50° C pendant 5 heures, sous pression initiale de 4000 kPa. Après refroidissement, on filtre le catalyseur sur lit de clarcel DIC et on évapore sous pression réduite (5,2 kPa).

On recristallise le résidu d'évaporation dans 100 $cm^3$ d'un mélange d'eau et d'éthanol (90-10 en volumes) ; on obtient ainsi 7,25 d'acétylamino-2 acétylaminométhyl-2 bicyclo (2.2.1) heptane, sous forme de fins cristaux blancs qui fondent à 192-4° C.

L'acétylamino-2 cyano-2 bicyclo (2.2.1) heptane, mélange des racémiques exo et endo dans un rapport (4/1), peut être obtenu de la manière suivante :

On dissout 7,98 g d'amino-2 cyano-2 bicyclo (2.2.1) heptane, sous forme du mélange des racémiques exo et endo dans un rapport (4/1), dans 100 $cm^3$ d'éther éthylique et on ajoute 5,7 $cm^3$ d'anhydride acétique. Après 20 heures d'agitation à 20° C, on essore le précipité formé et on le lave par 3 fois 20 $cm^3$ d'éther éthylique.

On obtient 9,25 g d'acétylamino-2 cyano-2 bicyclo (2.2.1) heptane sous forme de fins cristaux blancs qui fondent à 152-3° C.

L'amino-2 cyano-2 bicyclo (2.2.1) heptane peut être obtenu quantitativement, sous forme d'une huile incolore cristallisant vers 0° C et correspondant au mélange des racémiques exo et endo dans un rapport (4/1), par neutralisation de son chlorhydrate. Ce chlorhydrate peut être obtenu dans les conditions décrites par H.S. TAGER et coll., J. Amer. Chem. Soc., 94(3), 968-972 (1972).

## EXEMPLE 2

On met en suspension, sous atmosphère d'azote et à l'abri de la lumière, 0,81 g de cis (amino-2 aminométhyl-2 bicyclo (2.2.1) heptane) dichloro platine, sous forme du mélange des racémiques exo et endo dans un rapport (4/1), dans 2 $cm^3$ de méthanol et 18 $cm^3$ d'eau, on ajoute alors 0,68 g de nitrate d'argent dans 6,8 $cm^3$ d'eau. On agite 3 heures à 20° C, le chlorure d'argent formé est filtré sur verre fritté et le filtrat est ensuite clarifié sur une cartouche filtrante de porosité 3 $\mu$. La solution de dinitrate d'(amino-2 aminométhyl-2 bicyclo (2.2.1) heptane) diaqua platine est percolée au travers d'une colonne, de diamètre 1,2 cm, contenant 6 $cm^3$ de résine échangeuse d'ions Amberlite IRA 402 forme OH (0,0012 mole/$cm^3$). On ajoute, à la solution d'hydroxyde d'(amino-2 aminométhyl-2 bicyclo (2.2.1) octane) aqua hydroxo platine ainsi obtenue, une solution de 0,28 g d'acide phosphonoacétique dans 6 $cm^3$ d'eau.

On chromatographie sur une colonne, de 1 m de hauteur et de 2,5 cm de diamètre, contenant 120 g de Sephadex LH 20, en recueillant des fractions de 5 $cm^3$. On concentre à sec les fractions entre 415 et 455 $cm^3$.

On obtient ainsi 0,89 g de sel de sodium hexahydrate de cis (amino-2 aminométhyl-2 bicyclo (2.2.1) heptane) phosphonoacétato platine, mélange des racémiques exo et endo dans un rapport (4/1), sous forme de cristaux blancs brillants qui fondent avec décomposition à 314 °C.

EXEMPLE 3

On opère comme à l'exemple 2, à partir de 0,81 g de cis (amino-2 aminométhyl-2 bicyclo (2.2.1) heptane) dichloro platine, sous forme du mélange des racémiques exo et endo dans un rapport (4/1), dans 2 $cm^3$ de méthanol et 18 $cm^3$ d'eau, 0,68 g de nitrate d'argent dans 6,8 $cm^3$ d'eau. Après filtration du chlorure d'argent formé et clarification sur une cartouche de porosité 3 $\mu$, on ajoute à la solution de dinitrate d'(amino-2 aminométhyl-2 bicyclo (2.2.1) heptane) diaqua platine une solution de 0,35 g d'acide diméthyl-2,2 phosphonoacétique dans 6 $cm^3$ d'une solution 1 N d'hydroxyde de sodium dans l'eau. On chromatographie sur une colonne de 2,5 cm de diamètre contenant 120 g de Sephadex LH 20, en recueillant des fractions de 5 $cm^3$. On concentre à sec les fractions entre 410 et 465 $cm^3$.

On obtient ainsi 0,89 g de sel de sodium pentahydrate de cis (amino-2 aminométhyl-2 bicyclo (2.2.1) heptane (diméthyl-2,2 phosphonoactétato) platine, mélange des racémiques exo et endo dans un rapport (4/1), sous forme de cristaux blancs brillants qui fondent avec décomposition à 311 °C.

L'acide diméthyl-2,2 phosphonoacétique peut être préparé selon les conditions décrites dans la demande de brevet EP 290169.

EXEMPLE 4

On opère comme à l'exemple 1, à partir d'une solution de 3 g de dichlorhydrate d'amino-2 aminométhyl-2 adamantane dans 3 $cm^3$ de méthanol et 24 $cm^3$ d'une solution 1N d'hydroxyde de sodium dans l'eau, et d'une solution de 4,98 g de tétrachloroplatinate de potassium dans 30 $cm^3$ d'eau.

On obtient ainsi 3,9 g de cis (amino-2 aminométhyl-2 adamantane) dichloro platine, sous forme d'une poudre jaune qui fond avec décomposition à 360 °C.

Le dichlorhydrate d'amino-2 aminométhyl-2 adamantane peut être obtenu de la manière suivante :

On porte à reflux 6,45 g d'acétylamino-2 acétylaminométhyl-2 adamantane dans 180 $cm^3$ d'une solution 6 N d'acide chlorhydrique dans l'eau pendant 6 heures. On concentre à sec sous pression réduite (5,2 kPa), on reprend le résidu d'évaporation par 100 $cm^3$ d'éthanol et on ajoute, goutte à goutte 100 $cm^3$ d'éther isopropylique.

Le précipité obtenu est essoré sur verre fritté, lavé par 20 $cm^3$ d'un mélange d'éthanol et d'éther isopropylique (50-50 en volumes) et séché en étuve à 80 °C. On obtient ainsi 3,73 g de dichlorhydrate d'amino-2 aminométhyl-2 adamantane sous forme de cristaux blancs qui fondent à 300 °C.

L'acétylamino-2 acétylaminométhyl-2 adamantane peut être préparé de la manière suivante :

On charge, dans un autoclave de 500 cm3, une solution de 6,8 g d'acétylamino-2 cyano-2 adamantane dans 90 cm3 d'acide acétique et on ajoute 5,8 cm3 d'anhydride acétique puis 0,68 g d'oxyde de platine d'Adams. On soumet à l'hydrogénation catalytique, à 50 °C pendant 5 heures, sous une pression initiale de 4000 kPa. Après refroidissement, on filtre le catalyseur sur lit de clarcel DIC et on évapore sous pression réduite (5,2 kPa). Le résidu d'évaporation est recristallisé dans 100 cm3 d'eau.

On obtient ainsi 6,95 g d'acétylamino-2 acétylaminométhyl-2 adamantane, sous forme de fins cristaux blancs qui fondent à 108 °C.

L'acétylamino-2 cyano-2 adamantane peut être obtenu de la manière suivante :

On dissout 10 g d'amino-2 cyano-2 adamantane dans 100 $cm^3$ d'éther éthylique et on ajoute 5,8 cm3 d'anhydride acétique. Après 20 heures d'agitation à 20 °C, on essore le précipité formé et on le lave par 3 fois 20 cm3 d'éther éthylique.

On obtient ainsi 6,83 g d'acétylamino-2 cyano-2 adamantane, sous forme de fins cristaux blancs qui fondent à 140 °C.

L'amino-2 cyano-2 adamantane peut être obtenu de la manière suivante

On dissout 6,61 g de cyanure de sodium et 14,44 g de chlorure d'ammonium dans 150 cm3 d'eau et 250 cm3 d'éthanol. On ajoute 30 cm3 d'une solution d'ammoniaque à 28 % dans l'eau, puis, par portions, 20 g d'adamantanone-2. On porte à reflux pendant 5 heures. Après refroidissement à 0 °C, on acidifie à pH=1, par addition d'une solution 2 N d'acide chlorhydrique dans l'eau. L'adamantanone n'ayant pas réagi est essorée sur verre fritté et le filtrat est neutralisé à pH=9, par addition d'une solution 2N d'hydroxyde de sodium dans l'eau. Le précipité est essoré sur verre fritté, lavé par 5 fois 20 cm3 d'eau et séché sous

pression réduite (2,5 kPa).

On obtient ainsi 16,1 g d'amino-2 cyano-2 adamantane, sous forme de cristaux blancs qui fondent à 215° C.

EXEMPLE 5

On opère comme à l'exemple 1, à partir de 1,31 g de dichlorhydrate d'amino-2 aminométhyl-2 bicyclo (3.2.1) octane, sous forme du mélange des racémiques exo et endo dans un rapport (8/1), dans 3 $cm^3$ de méthanol et 12 $cm^3$ d'une solution 1 N d'hydroxyde de sodium dans l'eau, et de 2,46 g de tétrachloroplatinate de potassium en solution dans 12 $cm^3$ d'eau. On obtient ainsi 1,23 g d'hydrate de cis (amino-2 aminométhyl-2 bicyclo (3.2.1) octane) dichloro platine, mélange des racémiques exo et endo dans un rapport (8/1), sous forme d'une poudre jaune-beige qui fond avec décomposition à 326° C.

Le dichlorhydrate d'amino-2 aminométhyl-2 bicyclo (3.2.1) octane, mélange des racémiques exo et endo dans un rapport (8/1), peut être obtenu de la manière suivante :

On opère comme à l'exemple 1, mais à partir de 4,14 g d'acétylamino-2 acétylaminométhyl-2 bicyclo (3.2.1) octane, sous forme du mélange des racémiques exo et endo dans un rapport (8/1), dans 100 $cm^3$ d'une solution 6 N d'acide chlorhydrique dans l'eau pendant 18 heures au reflux ; en reprenant le résidu d'évaporation par 30 $cm^3$ d'éthanol et 100 $cm^3$ d'éther éthylique ; on obtient 3,1 g de dichlorhydrate d'amino-2 aminométhyl-2 bicyclo (3.2.1) octane sous forme de cristaux blancs qui fondent à 284-6° C.

L'acétylamino-2 acétylaminométhyl-2 bicyclo (3.2.1) octane, mélange des racémiques exo et endo dans un rapport (8/1), peut être obtenu de la manière suivante :

On opère à l'exemple 1, mais à partir de 9 g d'acétylamino-2 cyano-2 bicyclo (3.2.1) octane, sous forme du mélange des racémiques exo et endo dans un rapport (8/1), dans 140 $cm^3$ d'acide acétique et 8,8 $cm^3$ d'anhydride acétique, en présence de 0,9 g d'oxyde de platine d'Adams ; en chromatographiant le résidu d'évaporation sur une colonne de diamètre 5,5 cm contenant 250 g de gel de silice, en éluant par un mélange d'acétate d'éthyle et d'éthanol (75-25 en volumes) et en recueillant des fractions de 100 $cm^3$. Les fractions entre 1400 et 3100 $cm^3$ sont concentrées à sec. On obtient ainsi 9,75 g d'acétylamino-2 acétylaminométhyl-2 bicyclo (3.2.1) octane, sous forme de fins cristaux blancs qui fondent à 92-3° C.

L'acétylamino-2 cyano-2 bicyclo (3.2.1) octane, mélange des racémiques exo et endo dans un rapport (8/1), peut être obtenu de la manière suivante :

On opère comme à l'exemple 1, mais à partir de 8,05 g d'amino-2 cyano-2 bicyclo (3.2.1) octane, sous forme du mélange des racémiques exo et endo dans un rapport (8/1), dans 150 $cm^3$ d'éther éthylique et 5,6 $cm^3$ d'anhydride acétique ; on obtient 10,16 g d'acétylamino-2 cyano-2 bicyclo (3.2.1) octane sous forme de fins cristaux blancs qui fondent à 78-80° C.

L'amino-2 cyano-2 bicyclo (3.2.1) octane, mélange des racémiques exo et endo dans un rapport (8/1), peut être obtenu quantitativement à partir de son chlorhydrate, lui même préparé de la manière suivante :

On dissout 30 g de bicyclo (3.2.1) octanone-2 dans 300 $cm^3$ d'éthanol et on ajoute une solution de 18,2 g de cyanure de potassium et de 13,9 g de chlorure d'ammonium dans 300 $cm^3$ d'eau. Après 6 jours d'agitation à 20° C, l'éthanol est concentré sous pression réduite (5,2 kPa) et la phase aqueuse est extraite par 5 fois 200 $cm^3$ d'éther éthylique. La phase éthérée est lavée par 2 fois 50 $cm^3$ d'eau, séchée sur sulfate de sodium et concentrée sous pression réduite (5,2 kPa) jusqu'à un volume de 400 $cm^3$. On fait alors barboter de l'acide chlorhydrique gazeux jusqu'à saturation. Le précipité formé est essoré, lavé par 3 fois 25 $cm^3$ d'éther éthylique et séché sous pression réduite (2,5 kPa). On obtient ainsi 13,25 g de chlorhydrate d'amino-2 cyano-2 bicyclo (3.2.1) octane, mélange des racémiques exo et endo dans un rapport (8/1), sous forme de fins cristaux blancs qui fondent avec décomposition à 202-5° C.

EXEMPLE 6

On opère comme à l'exemple 1, à partir de 1,14 g de dichlorhydrate d'exo amino-3 aminométhyl-3 bicyclo (3.2.1) octane dans 10 $cm^3$ de méthanol et 10 $cm^3$ d'une solution 1 N d'hydroxyde de sodium dans l'eau, et de 2,08 g de tétrachloroplatinate de potassium en solution dans 21 $cm^3$ d'eau.

On obtient ainsi 1,32 g de cis (exo amino-3 aminométhyl-3 bicyclo (3.2.1) octane) dichloro platine, sous forme d'une poudre jaune qui fond avec décomposition à 332° C.

Le dichlorhydrate d'exo amino-3 aminométhyl-3 bicyclo (3.2.1) octane peut être obtenu de la manière suivante :

On opère comme à l'exemple 1, mais à partir de 4 g d'exo acétylamino-3 acétylaminométhyl-3 bicyclo

(3.2.1) octane dans 100 $cm^3$ d'une solution 6 N d'acide chlorhydrique dans l'eau pendant 18 heures au reflux ; en reprenant le résidu d'évaporation par 50 $cm^3$ d'éthanol et 100 $cm^3$ d'éther éthylique ; on obtient 2,15 g de dichlorhydrate d'exo amino-3 aminométhyl-3 bicyclo (3.2.1) octane, sous forme de cristaux blancs qui fondent avec décomposition à 296-8° C.

L'exo acétylamino-3 acétylaminométhyl-3 bicyclo (3.2.1) octane peut être obtenu de la manière suivante :

On opère comme à l'exemple 1, mais à partir de 4 g d'exo acétylamino-3 cyano-3 bicyclo (3.2.1) octane dans 60 $cm^3$ d'acide acétique et 3,8 $cm^3$ d'anhydride acétique, en présence de 0,5 g d'oxyde de platine d'Adams ; en concentrant à sec ; en chromatographiant sur une colonne de diamètre 5,5 cm contenant 300 g de gel de silice, en éluant par un mélange d'acétate d'éthyle et d'éthanol (85-15 en volumes) et en recueillant des fractions de 100 $cm^3$. Les fractions entre 1200 et 1800 $cm^3$ sont concentrées à sec, on obtient ainsi 4,1 g d'exo acétylamino-3 acétylaminométhyl-3 bicyclo (3.2.1) octane, sous forme d'une huile incolore.

Spectre de RMN (200 MHz, DMSO, δ en ppm)
8,00 (t, $-CH_2-\underline{NH}-CO-CH_3$)
7,40 (s, $-NH-CO-CH_3$)
3,30 (d, $-\underline{CH_2}-NH-CO-CH_3$)
1,65 et 1,82 (2s, $-CO-CH_3$ et $-CO-CH_3$)
1,25 à 1,60 et 1,90 à 2,15 (massifs, 12 H du cycle)

L'exo acétylamino-3 cyano-3 bicyclo (3.2.1) octane peut être obtenu de la manière suivante :

On opère comme à l'exemple 1, mais à partir de 10 g d'exo amino-3 cyano-3 bicyclo (3.2.1) octane dans 100 $cm^3$ d'éther éthylique et 10 $cm^3$ d'anhydride acétique ; en concentrant à sec ; en chromatographiant le résidu sur une colonne de 8 cm de diamètre contenant 500 g de gel de silice, en éluant par un mélange de dichlorométhane et d'acétate d'éthyle (90-10 en volumes) et en recueillant des fractions de 100 $cm^3$. Les fractions entre 1400 et 3000 $cm^3$ sont concentrées à sec, on obtient 8,69 g d'exo acétylamino-3 cyano-3 bicyclo (3.2.1) octane, sous forme d'une huile incolore visqueuse.

Spectre de RMN (200 MHz, DMSO, δ en ppm)
8,35 (s, $NH-CO-CH_3$)
1,90 (s, $-CO-CH_3$)
1,40 à 2,05 et 2,35 à 2,45 (massifs, 12 H du cycle)

L'exo amino-3 cyano-3 bicyclo (3.2.1) octane peut être obtenu dans les conditions décrites par H. CHRISTENSEN et coll., J. Med. Chem., 26, 1374-8 (1983).

## EXEMPLE 7

On opère comme à l'exemple 1, à partir de 2,27 g de dichlorhydrate d'amino-2 aminométhyl-2 bicyclo (2.2.2) octane dans 15 $cm^3$ de méthanol et 20 $cm^3$ d'une solution 1 N d'hydroxyde de sodium dans l'eau, et de 4,15 g de tétrachloroplatinate de potassium en solution dans 42 $cm^3$ d'eau.

On obtient ainsi 1,93 g de cis (amino-2 aminométhyl-2 bicyclo (2.2.2) octane) dichloro platine, sous forme d'une poudre jaune-beige qui fond avec décomposition à 317° C.

Le dichlorhydrate d'amino-2 aminométhyl-2 bicyclo (2.2.2) octane, peut être obtenu de la manière suivante :

On opère comme à l'exemple 1, mais à partir de 4,6 g d'acétylamino-2 bicyclo (2.2.2) octane, dans 100 $cm^3$ d'une solution 6 N d'acide chlorhydrique dans l'eau pendant 48 heures au reflux ; en reprenant le résidu d'évaporation par 25 $cm^3$ d'éthanol et 75 $cm^3$ d'éther éthylique ; on obtient 3 g de dichlorhydrate d'amino-2 aminométhyl-2 bicyclo (2.2.2) octane sous forme de cristaux blancs qui fondent à 302-303° C.

L'acétylamino-2 acétylaminométhyl-2 bicyclo (2.2.2) octane, peut être obtenu de la manière suivante :

On opère comme à l'exemple 1, mais à partir de 5,8 g d'acétylamino-2 cyano-2 bicyclo (2.2.2) octane, dans 100 $cm^3$ d'acide acétique et 5,6 $cm^3$ d'anhydride acétique, en présence de 0,6 g d'oxyde de platine d'Adams ; en chromatographiant le résidu d'évaporation sur une colonne de diamètre 5,5 cm contenant 200 g de gel de silice, en éluant par un mélange d'acétate d'éthyle et d'éthanol (75-25 en volumes) et en recueillant des fractions de 50 $cm^3$. Les fractions entre 800 et 1750 $cm^3$ sont concentrées à sec, on obtient ainsi 5,45 g d'acétylamino-2 acétylaminométhyl-2 bicyclo (2.2.2) octane, sous forme d'une huile visqueuse jaune pâle.

Spectre de RMN (200 MHz, DMSO, δ en ppm)
7,35 (t, $-CH_2-NH-CO-CH_3$)
6,30 (s, $-\underline{NH}-CO-CH_3$)

3,45 (d, $-CH_2-NH-CO-CH_3$)
1,82 et 1,98 (2s, $-CO-CH_3$ et $-CO-CH_3$)
1,35 à 2,25 (massifs 12 H du cycle)

L'acétylamino-2 cyano-2 bicyclo (2.2.2) octane peut être obtenu de la manière suivante :

On dissout 9,92 g de bicyclo (2.2.2) octanone dans 100 cm$^3$ d'éther éthylique et on ajoute 0,5 g d'iodure de zinc, puis à la suspension obtenue, une solution de 10 g de cyanure de triméthylsilyle dans 20 cm$^3$ d'éther éthylique. L'agitation est poursuivie pendant 30 minutes entre 20 et 25 °C, puis on ajoute 120 cm$^3$ d'une solution, saturée à 0 °C, d'ammoniac dans du méthanol et on porte la suspension obtenue au reflux pendant 4 heures. Les solvants sont évaporés sous pression réduite (5,2 kPa), le résidu est repris par 150 cm$^3$ d'éther éthylique, additionné de 12 cm$^3$ d'anhydride acétique et agité pendant 16 heures à 20 °C. Après concentration des solvants, le résidu est chromatographié sur une colonne de 10 cm de diamètre contenant 1500 g de gel de silice, en éluant par un mélange de dichlorométhane et d'acétate d'éthyle (90-10 pendant 5000 cm$^3$ puis 50-50 en volumes) et en recueillant des fractions de 50 cm$^3$. Les fractions entre 6050 et 6450 cm$^3$ sont concentrées à sec, on obtient ainsi 5,9 g d'acétylamino-2 cyano-2 bicyclo (2.2.2) octane, sous forme d'une huile visqueuse jaune pâle.

Spectre de RMN (300 MHz, $CDCl_3$, $\delta$ en ppm)
5,9 (s, $-NH-CO-CH_3$)
2,05 (s, $-CO-CH_3$)
1,6 à 2,5 (massif, 12 H du cycle)

La bicyclo (2.2.2) octanone peut être préparée dans les conditions décrites par B. ROCHE et coll., J. Org. Chem., 49(21), 3881-7 (1984).

EXEMPLE 8

On opère comme à l'exemple 1, à partir de 0,37 g de dichlorhydrate d'amino-8 aminométhyl-8 tricyclo (5.2.1.0/2,6) décane, sous forme de racémique endo, dans 5 cm$^3$ de méthanol et 3 cm$^3$ d'une solution 1 N d'hydroxyde de sodium dans l'eau, et de 0,61 g de tétrachloroplatine de potassium en solution dans 6 cm$^3$ d'eau.

On obtient ainsi 0,42 g de cis (amino-8 aminométhyl-8 tricyclo (5.2.1.0/2,6) décane) dichloro platine, racémique endo, sous forme d'une poudre jaune-beige qui fond avec décomposition à 314 °C.

L'amino-8 aminométhyl-8 tricyclo (5.2.1.0/2,6) décane, sous forme de racémique endo, peut être obtenu de la manière suivante :

On met en suspension 0,40 g d'amino-8 tricyclo (5.2.1.0/2,6) décylcarboxamide-8, sous forme de racémique endo, dans 30 cm$^3$ de tétrahydrofuranne anhydre, et on ajoute, goutte à goutte, 5 cm$^3$ d'une solution 2 N de complexe "borane-sulfure de diméthyle" dans le tétrahydrofuranne. La solution obtenue est portée au reflux pendant 20 heures ; après refroidissement on ajoute 10 cm$^3$ d'une solution 2 N d'acide chlorhydrique dans l'éther éthylique. Après concentration à sec, la poudre obtenue est dissoute dans 5 cm$^3$ d'eau et on amène à pH 10, par addition d'hydroxyde de sodium en solution 4 N dans l'eau, en présence de 10 cm$^3$ de dichlorométhane. Après décantation, la phase aqueuse est réextraite par 2 fois 5 cm$^3$ de dichlorométhane, les phases organiques jointes sont lavées par 5 cm$^3$ d'eau, séchées sur sulfate de sodium et concentrées à sec sous pression réduite (5,2 kPa).

L'huile incolore obtenue est dissoute dans 5 cm$^3$ d'éthanol et on ajoute 5 cm$^3$ d'une solution 2 N d'acide chlorhydrique dans l'éther éthylique. Le précipité formé est essoré et lavée par 2 fois 2 cm$^3$ d'éther éthylique. On obtient ainsi 0,44 g de dichlorhydrate d'amino-8 aminométhyl-8 tricyclo (5.2.1.0/2,6) décane, racémique endo, sous forme de cristaux blancs qui fondent avec décomposition à 282-285 °C.

L'amino-8 tricyclo (5.2.1.0/2,6) décylcarboxamide-8, sous forme de racémique endo, peut être obtenu de la manière suivante :

On opère comme à l'exemple 11, mais à partir d'une suspension de 3,12 g de benzyloxycarbonylamino-8 tricyclo (5.2.1.0/2,6) décylcarboxamide-8, sous forme de racémique endo, dans 300 cm$^3$ d'éthanol et 12 cm$^3$ d'une solution 1 N d'acide chlorhydrique dans l'eau, en présence de 0,5 g de palladium sur charbon à 10 % et sous une pression de 140 kPa d'hydrogène pendant 4 heures à 50 °C. Après filtration du catalyseur et concentration à sec, en reprenant le résidu par 100 cm$^3$ d'eau, en baséifiant à pH 10 et en extrayant au dichlorométhane, on obtient ainsi 1,62 g d'amino-8 tricyclo (5.2.1.0/2,6) décylcarboxamide-8, sous forme de paillettes blanches brillantes qui fondent à 131 °C.

Le benzyloxycarbonylamino-8 tricyclo (5.2.1.0/2,6) décylcarboxamide-8, racémique endo, est préparé de la manière suivante :

On opère comme à l'exemple 11, mais à partir de 4,8 g d'acide benzyloxycarbonylamino-8 tricyclo

(5.2.1.0/2,6) décylcarboxylique-8, sous forme de racémique endo, dans 150 $cm^3$ de tétrahydrofuranne anhydre et 2,05 $cm^3$ de triéthylamine anhydre ; de 2,1 $cm^3$ de chloroformiate d'isobutyle, puis d'un barbotage d'ammoniac. Le précipité formé est versé sur 200 $cm^3$ d'eau, essoré, lavé par 3 fois par 50 $cm^3$ d'eau et séché en étuve à 80 °C. On obtient ainsi 3,11 g de benzyloxycarbonylamino-8 tricyclo (5.2.1.0/2,6) décylcarboxamide-8, sous forme d'une poudre blanche qui fond à 148 °C.

On peut obtenir l'acide benzyloxycarbonylamino-8 tricyclo (5.2.1.0/2,6) décylcarboxylique-8, racémique endo, de la manière suivante :

On opère comme à l'exemple 11, mais à partir de 5,3 g d'acide amino-8 tricyclo (5.2.1.0/2,6) décylcarboxylique-8, sous forme d'un mélange des racémiques endo et exo dans un rapport (6/1), dans 60 $cm^3$ d'une solution 1 N d'hydroxyde de sodium dans l'eau et de 3,85 $cm^3$ de chloroformiate de benzyle. En extrayant l'excès de chloroformiate de benzyle à l'éther éthylique, puis en acidifiant la phase aqueuse à pH 1 et en extrayant l'huile, qui décante alors, au dichlorométhane ; en recristallisant, après concentration, le résidu huileux dans 250 $cm^3$ d'un mélange d'eau et d'éthanol (30-70 en volumes), on obtient alors 4,98 g d'acide benzyloxycarbonylamino-8 tricyclo (5.2.1.0/2,6) décylcarboxylique-8, racémique endo, sous forme d'une poudre blanche qui fond à 122 °C.

L'acide amino-8 tricyclo (5.2.1.0/2,6) décylcarboxylique-8, sous formes d'un mélange des racémiques endo et exo dans un rapport (6/1) peut être obtenu dans les conditions décrites par J.R. SUFRIN et coll., Mol. Pharmacol., 15, 661-677 (1979).

## EXEMPLE 9

On opère comme à l'exemple 1, à partir de 0,80 g de dichlorhydrate d'amino-8 aminométhyl-8 tricyclo (5.2.1.0/2,6) décane, sous forme de racémique exo, dans 5 $cm^3$ de méthanol et 6 $cm^3$ d'une solution 1 N d'hydroxyde de sodium dans l'eau, et de 1,24 g de tétrachloroplatine de potassium en solution dans 12 $cm^3$ d'eau.

On obtient ainsi 0,85 g de cis (amino-8 aminométhyl-8 tricyclo (5.2.1.0/2,6) décane) dichloro platine, racémique exo, sous forme d'une poudre jaune-beige qui fond avec décomposition à 318 °C.

L'amino-8 aminométhyl-8 tricyclo (5.2.1.0/2,6) décane, sous forme racémique exo, peut être obtenu de la manière suivante :

On opère comme à l'exemple 1, mais à partir de 1,97 g d'acétylamino-8 acétylaminométhyl-8 tricyclo (5.2.1.0/2,6) décane, sous forme de racémique exo, dans 50 $cm^3$ d'une solution 6 N d'acide chlorhydrique dans l'eau pendant 24 heures au reflux. En reprenant le résidu d'évaporation par 20 $cm^3$ d'éthanol et 80 $cm^3$ d'éther éthylique, on obtient 1,26 g de dichlorhydrate d'amino-6 aminométhyl-8 tricyclo (5.2.1.0/2,6) décane, racémique exo, sous forme de cristaux blancs qui fondent à 273-5 °C.

L'acétylamino-8 acétylaminométhyl-8 tricyclo (5.2.1.0/2,6) décane, sous forme de racémique exo, peut être obtenu de la manière suivante :

On opère comme à l'exemple 1, mais à partir de 5,8 g d'acétylamino-8 cyano-8 tricyclo (5.2.1.0/2,6) décane, racémique exo, dans 100 $cm^3$ d'acide acétique et 5,6 $cm^3$ d'anhydride acétique, en présence de 0,6 g d'oxyde de platine d'Adams ; en recristallisant le résidu d'évaporation dans 200 $cm^3$ d'un mélange d'éthanol et d'eau (25-75 en volumes), on obtient 4,33 g d'acétylamino-8 cyano-8 tricyclo (5.2.1.0/2,6) décane, racémique exo, sous forme de cristaux blancs brillants qui fondent à 204 °C.

L'acétylamino-8 cyano-8 tricyclo (5.2.1.0/2,6) décane, sous forme de racémique exo, peut être obtenu de la manière suivante :

On opère comme à l'exemple 1, mais à partir de 4 g d'amino-8 cyano-8 tricyclo (5.2.1.0/2,6) décane, sous forme de racémique exo, dans 100 $cm^3$ d'éther éthylique et 3,85 $cm^3$ d'anhydride acétique ; on obtient 4,35 g d'acétylamino-8 cyano-8 tricyclo (5.2.1.0/2,6) décane, racémique exo, sous forme de fins cristaux blancs qui fondent à 141 °C.

L'amino-8 cyano-8 tricyclo (5.2.1.0/2,6) décane, sous forme de racémique exo, est obtenu quantitativement à partir de son chlorhydrate, qui peut être obtenu, sous forme de racémique exo pur, dans les conditions décrites par J.R. SUFRSN et coll., Mol. Pharmacol., 15, 661-677 (1979).

## EXEMPLE 10

On porte, à 80 °C pendant 15 minutes, une solution de 2,07 g de tétrachloroplatinate de potassium et de 3,32 g d'iodure de potassium dans 20 $cm^3$ d'eau. On ajoute alors la solution de tétraiodoplatinate de potassium ainsi obtenue à une solution de 1,06 g de dichlorhydrate d'amino-2 aminométhyl-2 bicyclo

(2.2.1) heptane, sous forme de racémique exo (1R, 2R, 4S) et (1S, 2S, 4R), dans 5 $cm^3$ de méthanol et 10 $cm^3$ d'une solution 1 N d'hydroxyde de sodium dans l'eau. Après 3 heures d'agitation à 25°C à l'abri de la lumière, le précipité formé est essoré sur verre fritté, lavé 3 fois par 5 $cm^3$ d'eau puis par 5 $cm^3$ d'acétone, on obtient ainsi 2,67 g de complexe diiodo.

La totalité de ce complexe est mise en suspension dans 10 $cm^3$ de méthanol et 90 $cm^3$ d'eau, on ajoute 1,55 g de nitrate d'argent, puis on agite 4 heures à l'abri de la lumière. Après essorage de l'iodure d'argent formé, la solution est clarifiée sur une cartouche de porosité 3 $\mu$.

On ajoute, à la solution de dinitrate de diaqua platine ainsi obtenue, 1,01 g de chlorure de potassium et on agite 4 heures à l'abri de la lumière. Le précipité formé est essoré et lavé par 3 fois 10 $cm^3$ d'eau. On obtient ainsi 1,34 g de cis (amino-2 aminométhyl-2 bicyclo (2.2.1) heptane) dichloro platine, racémique exo, monohydrate, sous forme d'une poudre jaune-beige qui fond avec décomposition à 333°C.

Le dichlorhydrate d'amino-2 aminométhyl-2 bicyclo (2.2.1) heptane, racémique exo, peut être obtenu de la manière suivante :

On opère comme à l'exemple 3, mais à partir de 7,85 g d'acétylamino-2 acétylaminométhyl-2 bicyclo (2.2.1) heptane, sous forme de racémique exo, dans 250 $cm^3$ d'une solution 6 N d'acide chlorhydrique dans l'eau pendant 8 heures au reflux ; en reprenant le résidu d'évaporation par 100 $cm^3$ d'éthanol et 300 $cm^3$ d'éther éthylique ; on obtient 6,87 g de dichlorhydrate d'amino-2 aminométhyl-2 bicyclo (2.2.1) heptane sous forme de cristaux blancs qui fondent à 271°C.

L'acétylamino-2 acétylaminométhyl-2 bicyclo (2.2.1) heptane, racémique exo, peut être obtenu de la manière suivante :

On opère comme à l'exemple 3, mais à partir de 7,14 g d'acétylamino-2 cyano-2 bicyclo (2.2.1) heptane, sous forme de racémique exo, dans 250 $cm^3$ d'acide acétique et 7,5 $cm^3$ d'anhydride acétique, en présence 0,7 g d'oxyde de platine d'Adams ; en reprenant le résidu d'évaporation par 100 $cm^3$ d'eau et d'éthanol (80-20 en volumes) ; on obtient ainsi 8,7 g d'acétylamino-2 acétylaminométhyl-2 bicyclo (2.2.1) heptane, sous forme de fins cristaux blancs qui fondent à 208°C.

L'acétylamino-2 cyano-2 bicyclo (2.2.1) heptane, racémique exo, peut être obtenu de la manière suivante :

On opère comme à l'exemple 3, mais à partir de 6,33 g d'amino-2 cyano-2 bicyclo (2.2.1) heptane, sous forme du mélange des racémiques exo et endo dans un rapport (8/1), dans 100 $cm^3$ d'éther éthylique et 4,7 $cm^3$ d'anhydride acétique ; on obtient, après recristallisation dans 300 $cm^3$ d'un mélange d'éthanol et d'eau (15-85 en volumes), 7,14 g d'acétylamino-2 cyano-2 bicyclo (2.2.1) heptane sous forme de fins cristaux blancs qui fondent à 164°C.

En opérant dans les conditions décrites par H.S. TAGER et coll., J. Amer. Chem. Soc., 94(3), 968-972 (1972), après deux recristallisations successives dans le minimum d'acide chlorhydrique en solution aqueuse 1 N, on obtient le chlorhydrate de l'amino-2 cyano-2 bicyclo (2.2.1) heptane fondant à 232-233°C.

L'amino-2 cyano-2 bicyclo (2.2.1) heptane peut être obtenu quantitativement, sous forme d'une huile incolore cristallisant vers 20°C et correspondant au mélange des racémiques exo et endo dans un rapport (8/1), par neutralisation de son chlorhydrate.

EXEMPLE 11

On dissout, à l'abri de la lumière, 0,098 g d'amino-2 aminométhyl-2 bicyclo (2.2.1) heptane, sous forme de racémique endo, dans 10 $cm^3$ de méthanol et 15 $cm^3$ d'eau. On ajoute une solution de 0,258 g de trichloro(éthylène)platinate de potassium (sel de Zeise) dans 5 $cm^3$ de méthanol. On chauffe à 35°C pendant 3 heures, puis on maintient l'agitation pendant 20 heures à 20°C. Le précipité formé est essoré, lavé par 2 fois 2,5 $cm^3$ d'un mélange de méthanol et d'eau (50-50 en volumes) puis par 2,5 $cm^3$ de méthanol. On obtient ainsi 0,150 g de cis (amino-2 aminométhyl-2 bicyclo (2.2.1) heptane) dichloro platine, racémique endo (1R, 2S, 4S) et (1S, 2R, 4R), dihydrate, sous forme d'une poudre jaune-beige qui fond avec décomposition à 322°C.

On peut obtenir l'amino-2 aminométhyl-2 bicyclo (2.2.1) heptane, racémique endo, de la manière suivante :

On dissout, sous azote, 0,308 g d'amino-2 bicyclo (2.2.1) heptylcarboxamide-2, sous forme de racémique endo, dans 25 $cm^3$ de tétrahydrofuranne anhydre et on ajoute 10 $cm^3$ d'une solution 1 N de diborane dans le tétrahydrofuranne anhydre. On porte au reflux 20 heures ; après refroidissement à 0°C on ajoute 20 $cm^3$ d'éthanol, puis avec précaution 5 $cm^3$ d'une solution 2 N d'acide chlorhydrique dans l'éther éthylique. Les solvants sont concentrés à sec sous pression réduite (5,2 kPa), le résidu est repris par 20 $cm^3$ d'eau, basifié à pH 10 par addition d'une solution 1 N d'hydroxyde de sodium dans l'eau, puis extrait

3 fois par 20 $cm^3$ de dichlorométhane. On obtient ainsi 0,195 g d'amino-2 aminométhyl-2 bicyclo (2.2.1) heptane, sous forme d'une huile jaune pâle ; cette diamine peut être caractérisée par son chlorhydrate, poudre blanche qui fond à 291 °C.

On peut obtenir l'amino-2 bicyclo (2.2.1) heptylcarboxamide-2, racémique endo, de la manière suivante :

On met en suspension 4,32 g de benzyloxycarbonylamino-2 bicyclo (2.2.1) heptylcarboxamide-2, sous forme de racémique endo, dans 150 $cm^3$ d'éthanol et 15 $cm^3$ d'une solution 1 N d'acide chlorhydrique dans l'eau, puis on ajoute 0,5 g de palladium sur charbon à 10 %. On soumet le mélange à une pression de 140 kPa d'hydrogène pendant 4 heures à 50 °C. Après refroidissement, filtration du catalyseur et concentration à sec du filtrat sous pression réduite (5,2 kPa), le résidu est repris par 100 $cm^3$ d'eau, baséifié pH 10 par addition d'une solution 2 N d'hydroxyde de sodium dans l'eau et extrait par 4 fois 100 $cm^3$ de dichlorométhane. On obtient ainsi 1,66 g d'amino-2 bicyclo (2.2.1) heptylcarboxamide-2, sous forme de paillettes blanches brillantes qui fondent à 178 °C.

Le benzyloxycarbonylamino-2 bicyclo (2.2.1) heptylcarboxamide-2, racémique endo, est préparé de la manière suivante :

On dissout 11,6 g d'acide benzylocarbonylamino-2 bicyclo (2.2.1) heptylcarboxylique-2, sous forme de racémique endo, dans 200 $cm^3$ de tétrahydrofuranne anhydre et 6,2 $cm^3$ de triéthylamine anhydre. Après refroidissement à -20 °C, on ajoute 5,7 $cm^3$ de chloroformiate d'isobutyle et on agite la suspension blanche obtenue pendant 1 heure entre -20 et 0 °C, puis on fait barboter, sous agitation, de l'ammoniac jusqu'à saturation à 0 °C. On maintient alors l'agitation pendant 16 heures à 20 °C, le précipité formé est versé sur 700 $cm^3$ d'eau, essoré, lavé par 3 fois par 50 $cm^3$ d'eau et séché en étuve à 80 °C. On obtient ainsi 9,5 g de benzyloxycarbonylamino-2 bicyclo (2.2.1) heptylcarboxamide-2, sous forme d'une poudre blanche qui fond à 188 °C.

L'acide benzyloxycarbonylamino-2 bicyclo (2.2.1) heptylcarboxylique-2, racémique endo, est préparé de la manière suivante :

On dissout 12,4 g d'acide amino-2 bicyclo (2.2.1) heptylcarboxylique-2, sous forme d'un mélange des racémiques endo et exo dans un rapport (12/1), dans 220 $cm^3$ d'une solution 1 N d'hydroxyde de sodium dans l'eau. Après refroidissement à -10 °C, on ajoute 18,3 $cm^3$ de chloroformiate de benzyle, puis on agite pendant 5 heures en laissant revenir la température de -10 à +20 °C. L'excès de chloroformiate de benzyle est alors extrait par 2 fois 100 $cm^3$ d'éther éthylique, la phase aqueuse est ensuite acidifiée à pH 1 par addition d'acide chlorhydrique concentré et l'huile, qui décante alors, est extraite par 3 fois 100 $cm^3$ de dichlorométhane. Après reconcentration du dichlorométhane sous pression réduite (5,2 kPa), le résidu huileux est recristallisé dans 300 $cm^3$ d'un mélange d'eau et d'éthanol (65-35 en volumes). On obtient ainsi 13,5 g d'acide benzyloxycarbonylamino-2 bicyclo (2.2.1) heptylcarboxylique-2, sous forme d'une poudre blanche qui fond à 129 °C.

L'acide amino-2 bicyclo (2.2.1) heptylcarboxylique-2, mélange des racémiques endo et exo dans un rapport (12/1) peut être obtenu dans les conditions décrites par H.N. CHRISTENSEN et coll., J. Biol. Chem., 244(6), 1510-20 (1969).

La présente invention concerne également les compositions pharmaceutiques qui contiennent comme produit actif au moins un produit de formule générale (I) à l'état pur (sous forme libre ou sous forme de sel) ou en association avec un ou plusieurs adjuvants pharmaceutiquement acceptables. Ces compositions peuvent être utilisées par voie parentérale.

Les compositions pour administration parentérale, peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui seront dissoutes au moment de l'emploi dans de l'eau stérile ou tout autre milieu stérile injectable.

En thérapeutique humaine, les médicaments selon la présente invention sont particulièrement utiles dans le traitement des cancers digestifs, des cancers pulmonaires, des cancers des testicules ou des ovaires ainsi que dans les traitements des cancers de la tête et du cou.

D'une façon générale, le médecin déterminera la posologie qu'il estime la plus appropriée en fonction de l'âge, du poids et de tous les autres facteurs propres au sujet à traiter.

Le mode d'administration préféré est la voie intra-veineuse. A titre indicatif, les médicaments selon l'invention peuvent être administrés chez l'homme à raison de 250 à 1000 $mg/m^2$ par traitement, par voie

intra-veineuse.

L'exemple suivant, donné à titre non limitatif, illustre une composition selon la présente invention :

Exemple -

On prépare un flacon de 50 cm3 contenant 835,3 mg d'hydrate de cis (amino-1 aminométhyl-1 bicyclo (3.2.1) octane) dichloro platine et une ampoule contenant 50 cm3 de sérum glucosé isotonique destinée à être ajoutée au flacon au moment de l'emploi.

La solution ainsi préparée qui contient 800 mg de cis (amino-1 aminométhyl-1 bicyclo (3.2.1) octane) dichloro platine est prête à être intégrée dans un liquide de perfusion.

**Revendications**

1 - Un nouveau complexe dérivé du platine de formule générale :

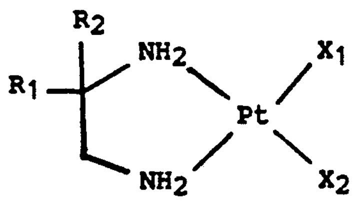

dans laquelle :
- $R_1$ et $R_2$ forment ensemble un carbocycle polycyclique saturé ou insaturé contenant 7 à 12 atomes de carbone, ou un hétérocycle saturé ou partiellement saturé, mono, bi ou tricyclique contenant 5 à 11 chaînons et dont l'hétéroatome est choisi parmi l'oxygène, le soufre ou l'azote. Ce dernier pouvant être éventuellement substitué par un radical alcoyloxycarbonyle et
- $X_1$ et $X_2$ représentent des atomes de chlore ou forment ensemble
- soit un radical de structure :

$$-O-\underset{\underset{O}{\|}}{C}-(CR_6R_7)_n-\underset{\underset{O}{\|}}{C}-O-$$

dans laquelle n égale 0 à 2 et $R_6$ et $R_7$ identiques ou différents sont des atomes d'hydrogène ou lorsque n égale 1 peuvent être des radicaux alcoyle ou former ensemble avec l'atome de carbone auquel ils sont attachés, un radical cyclobutyle,
- soit un radical de structure :

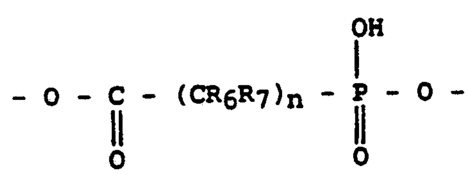

dans laquelle n, $R_6$ et $R_7$ sont définis comme ci-dessus, étant entendu que les radicaux et portions acoyle cités ci-dessus contiennent 1 à 4 atomes de carbone en chaîne droite ou ramifiée ainsi que ses hydrates et ses sels lorsqu'ils existent.

2 - Un nouveau complexe dérivé du platine selon la revendication 1, caractérisé en ce que $R_1$ et $R_2$ forment ensemble un carbocycle polycyclique saturé choisi parmi : bicyclo (2.2.1) heptane, bicyclo (3.2.1) octane, bicyclo (2.2.2) octane, bicyclo (3.2.2) nonane, bicyclo (3.3.1) nonane, adamantane, décahydronaphtalène, tétrahydronaphtalène, spiro (5.5) undécane, tricyclo (5.2.1.0/2,6) décane ou indane ou $R_1$ et $R_2$ forment ensemble un hétérocycle choisi parmi : chromanyle-4, coumaryle-3, homopipéridinyle-4, pipéridinyle-4, pyrrolidinyle-3, quinuclidinyle-3, tétrahydropyrannyle-4, tétrahydrofurannyle-3, tétrahydrothiopyrannyle-4, tétrahydrothiofurannyle-3 ou tétrahydroquinoléyle-4, ainsi que ses hydrates et ses sels.

3 - Un nouveau complexe dérivé du platine, selon la revendication 1, caractérisé en ce que $R_1$ et $R_2$

forment ensemble un carbocycle polycyclique choisi parmi : bicyclo (2.2.1) heptane, adamantane, bicyclo (3.2.1) octane, bicyclo (2.2.2) octane ou tricyclo (5.2.1.0/2,6) décane, et $X_1$ et $X_2$ représentent des atomes de chlore ou un radical de structure :

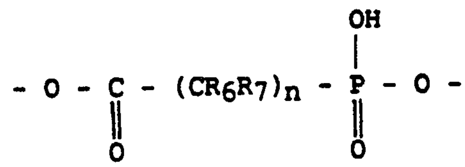

pour lequel n égale 1 et, $R_6$ et $R_7$ sont des atomes d'hydrogène ou des radicaux méthyle ou forment ensemble un radical cyclobutyle, ainsi que ses hydrates et ses sels.

4 - Le cis (amino-2 aminométhyl-2 bicyclo (2.2.1) heptane) dichloro platine ainsi que ses formes hydratées.

5 - Le cis (amino-2 aminométhyl-2 bicyclo (3.2.1) octane) dichloro platine ainsi que ses formes hydratées.

6 - Le cis (amino-2 aminométhyl-2 adamantane) dichloro platine ainsi que ses hydrates.

7 - Le cis (amino-3 aminométhyl-3 bicyclo (3.2.1) octane) dichloro platine ainsi que ses hydrates.

8 - Le cis (amino-2 aminométhyl-2 bicyclo (2.2.2) octane) dichloroplatine ainsi que ses hydrates.

9 - Un procédé de préparation d'un complexe dérivé du platine, selon la revendication 1, caractérisé en ce que l'on fait agir le tétrachloroplatinate de potassium sur une diamine de formule générale :

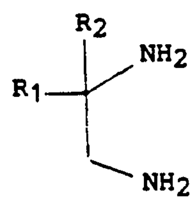

dans laquelle $R_1$ et $R_2$ sont définis comme dans la revendication 1, puis éventuellement, lorsque l'on veut obtenir un complexe selon la revendication 1 pour lequel $X_1$ et $X_2$ sont autres que des atomes de chlore, transforme le complexe chloré obtenu en un complexe dicarboxylé ou phosphonocarboxylé et le cas échéant transforme éventuellement le complexe phosphonocarboxylé en un sel.

10 - Un procédé de préparation d'un complexe dérivé du platine, selon la revendication 1, caractérisé en ce que l'on fait agir le tétrachloroplatinate de potassium sur une diamine selon la revendication 9, en présence d'un excès d'iodure de potassium, puis traite par le nitrate d'argent puis par un excès de chlorure alcalin le complexe diiodé obtenu intermédiairement, et, lorsque l'on veut obtenir un complexe selon la revendication 1 pour lequel $X_1$ et $X_2$ sont autres que des atomes de chlore, transforme le complexe dichloré obtenu en un complexe dicarboxylé ou phosphonocarboxylé et le cas échéant transforme éventuellement le complexe phosphonocarboxylé en un sel.

11- Un procédé de préparation d'un complexe dérivé du platine selon la revendication 1, caractérisé en ce que l'on fait réagir le trichloro(éthylène)platinate de potassium sur une diamine selon la revendication 9, puis lorsque l'on veut obtenir un complexe selon la revendication 1 pour lequel $X_1$ et $X_2$ sont autres que des atomes de chlore, transforme le complexe dichloré obtenu en un complexe dicarboxylé ou phosphonocarboxylé et le cas échéant transforme éventuellement le complexe phosphonocarboxylé en un sel.

12 - Un procédé selon l'une des revendications 9 à 11 caractérisé en ce que la transformation du complexe chloré obtenu, en un complexe dicarboxylé ou phosphonocarboxylé, s'effectue par l'intermédiaire du complexe dinitrate de diaqua de formule générale :

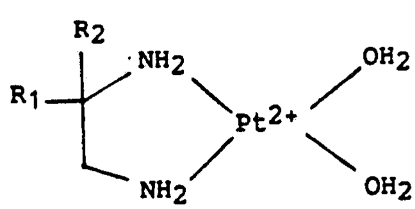 , $2NO_3^-$

dans laquelle $R_1$ et $R_2$ sont définis comme dans les revendications 9 à 11, sur lequel on fait agir un sel d'un acide de formule générale :

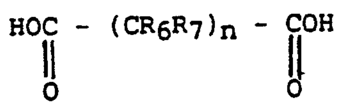

ou

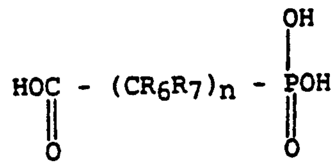

dans laquelle $R_6$, $R_7$ et n sont définis comme dans les revendications 2 à 6, puis le cas échéant libère le complexe phosphonocarboxylé de son sel et le transforme éventuellement en un autre sel.

13 - Un procédé selon l'une des revendications 9 à 11, caractérisé en ce que la transformation du complexe dichloré obtenu en un complexe dicarboxylé ou phosphonocarboxylé s'effectue par l'intermédiaire d'un complexe dinitrate de diaqua de formule générale :

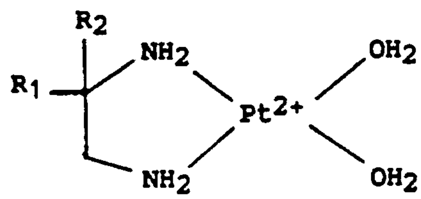

, $2NO_3^-$

dans laquelle $R_1$ et $R_2$ sont définis comme dans les revendications 9 à 11, puis passage sur une résine échangeuse d'anions (forme OH) et addition d'un acide de formule générale :

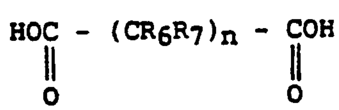

ou

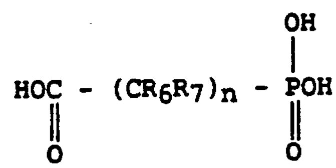

dans laquelle $R_6$, $R_7$ et n sont définis comme dans les revendications 9 à 11, puis transforme éventuellement le complexe obtenu en un sel.

14 - Composition pharmaceutique caractérisée en ce qu'elle contient au moins un complexe dérivé du platine selon la revendication 1, à l'état pur, éventuellement sous forme de sel ou d'hydrate ou en association avec tout diluant ou adjuvant compatible et pharmaceutiquement acceptable.

Revendication pour les Etats contractants suivants: ES,GR

Procédé de préparation d'un nouveau complexe dérivé du platine de formule générale :

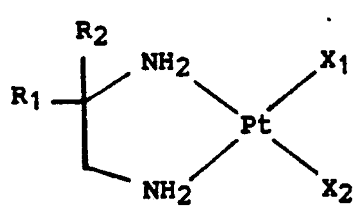

dans laquelle :

- $R_1$ et $R_2$ forment ensemble un carbocycle polycyclique saturé ou insaturé contenant 7 à 12 atomes de carbone, ou un hétérocycle saturé ou partiellement saturé, mono, bi ou tricyclique contenant 5 à 11 chaînons et dont l'hétéroatome est choisi parmi l'oxygène, le soufre ou l'azote. Ce dernier pouvant être éventuellement substitué par un radical alcoyloxycarbonyle et
- $X_1$ et $X_2$ représentent des atomes de chlore ou forment ensemble

- soit un radical de structure :

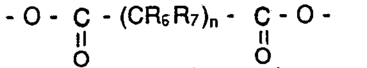

dans laquelle n égale 0 à 2 et $R_6$ et $R_7$ identiques ou différents sont des atomes d'hydrogène ou lorsque n égale 1 peuvent être des radicaux alcoyle ou former ensemble avec l'atome de carbone auquel ils sont attachés, un radical cyclobutyle,
- soit un radical de structure :

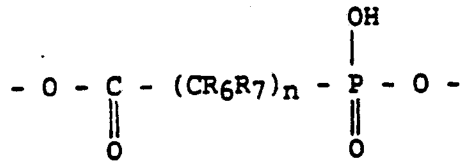

dans laquelle n, $R_6$ et $R_7$ sont définis comme ci-dessus, étant entendu que les radicaux et portions acoyle cités ci-dessus contiennent 1 à 4 atomes de carbone en chaîne droite ou ramifiée ainsi que ses hydrates et ses sels lorsqu'ils existent, caractérisé en ce que l'on fait agir le tétrachloroplatinate de potassium sur une diamine de formule générale :

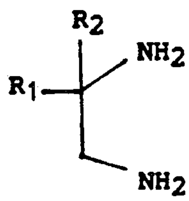

dans laquelle $R_1$ et $R_2$ sont définis comme précédemment ou bien,
caractérisé en ce que l'on fait agir le tétrachloroplatinate de potassium sur une diamine telle que définie ci-dessus en présence d'un excès d'iodure de potassium, puis traite par le nitrate d'argent puis par un excès de chlorure alcalin le complexe diiodé obtenu intermédiairement ou bien,
caractérisé en ce que l'on fait réagir le trichloro(éthylène)platinate de potassium sur une diamine telle que définie ci-dessus, puis, pour obtenir un complexe dicarboxylé ou phosphonocarboxylé transforme le complexe chloré obtenu par l'intermédiaire du complexe dinitrate de diaqua de formule générale :

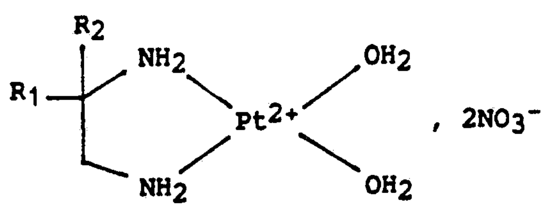

dans laquelle $R_1$ et $R_2$ sont définis comme précédemment, sur lequel on fait agir un sel d'un acide de formule générale :

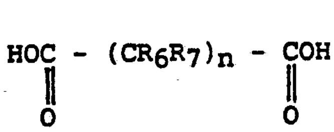

ou

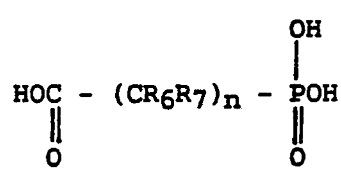

dans laquelle $R_6$, $R_7$ et n sont définis comme précédemment, puis le cas échéant libère le complexe phosphonocarboxylé de son sel et le transforme éventuellement en un autre sel, ou bien
transforme le complexe dichloré obtenu en un complexe dicarboxylé ou phosphonocarboxylé par l'intermédiaire d'un complexe dinitrate de diaqua de formule générale :

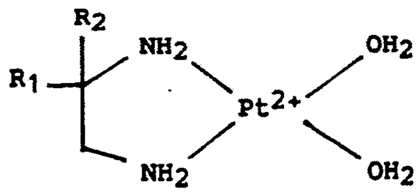

, $2NO_3^-$

dans laquelle $R_1$ et $R_2$ sont définis comme précédemment, puis passage sur une résine échangeuse d'anions (forme OH) et addition d'un acide de formule générale :

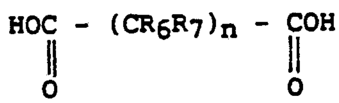

ou

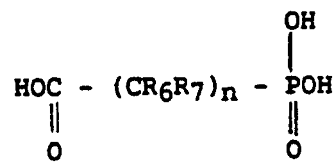

dans laquelle $R_6$, $R_7$ et n sont définis comme précédemment et transforme éventuellement le complexe obtenu en un sel.

Office européen des brevets

# RAPPORT DE RECHERCHE EUROPEENNE

Numero de la demande

EP 90 40 0703

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 155 705 (BRISTOL-MYERS COMPANY)<br>* revendications 10,12, exemple 15 * | 1,9 | C 07 F 15/00<br>A 61 K 31/28 |
| X | CHEMICAL ABSTRACTS vol. 109, no. 15, 10 octrobre 1988, abrégé no. 190186e, Columbus, Ohio, US; M. P. GEORGIADIS et al.: "Products from furans. V. Synthesis of oxygen-containing isosteres of sympathomimetic amines via 6-hydroxy-2H-pyran-3(6H)-ones and their cis-platinum(II) complexes" & J. Heterocycl. Chem. 1988, vol. 25, no. 3, pages 995-1002 | 1 | |
| A | US-A-4 739 087 (R.J. SPEER et al.)<br>* exemple 18 * | 1 | |
| D,A | EP-A-0 290 169 (ENGELHARD)<br>* revendication 1 * | 1 | |
| D,A | GB-A-2 148 891 (NIPPON KAYAKU KABUSHIKI KAISHA)<br>* revendication 1 * | 1 | **DOMAINES TECHNIQUES RECHERCHES (Int. Cl.5)**<br>C 07 F 15/00<br>A 61 K 31/28 |

Le présent rapport a été établi pour toutes les revendications

| Lieu de la recherche | Date d'achèvement de la recherche | Examinateur |
|---|---|---|
| BERLIN | 14-06-1990 | KAPTEYN H G |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant